# EUROPEAN PATENT APPLICATION

(11) **EP 2 128 595 A1**
(43) Date of publication of application: **02.12.2009**
(21) Application number: 09160694.7
(22) Date of filing: 19.05.2009
(51) Int. Cl.: G01N 1/12, G01N 1/20, G01N 15/04, G01N 33/18, G01N 1/10

(54) **Continual sediment sampler for small sloping flows**

(30) Priority: 29.05.2008 CZ 20080329
(71) Applicant: Výzkumný ústav vodohospodárský T.G. Masaryka v.v.i., 16062 Praha 6 (CZ)
(72) Inventor: Simon, Ondrej, 25268, Stredokluky (CZ); Douda, Karel, 39165, Cernýsovice-Hute (CZ)
(74) Representative: Novotny, Jaroslav

(57) **Abstract**

Continual sediment mobile sampler for sloping flows, composed of the settling vessel (3) of a cylindrical shape with a screw-on cap and two openings into which the suction and discharge pipes (2, 4) are fitted. The suction pipe is fitted in its intake part with a basket (1) and the outlet of the discharge pipe (4) must be at least 10 cm below the level of the suction basket (1). The sampler is adapted to the use in water flows, water channels or gravitational pipes, including one time use.

## Description

### TECHNICAL FIELDS

The technological solution relates to the collection of concentrated samples of fine suspended particles from water flows, water channels or gravitational pipes having a satisfactory gradient, including samples for the analysis of trace amounts of chemical substances and microbiological samples.

### BACKGROUND ARTS

Monitoring of chemical, biological and other properties of small suspensions occurring in the uplifted water environment has got shorter traditions than the observation of substances in solutions.

Collection of wash-loads, the fraction called in biology FPOM (fine particulate organic matter), tackles several problems. These relate mostly to concentration of too thin samples in very clean flows and the representation of fast time fluctuation of concentrations in small flows. Collection of a well representing sample, allowing for the calculation of wash-load in one litre of water, could be also a problem.

These complex operations are mostly resolved in very complicated analysing stations featuring continual sample collection or with mobile automatic programmable samplers. Their price and the impossibility of these facilities' use without supervision in localities without securing them in solid constructions fundamentally limit their use within more extensive area monitoring programmes. The CHMU network (Czech Institute of Hydrometeorology), for example, uses mostly manual collecting by an authorised person living in the place of interest even for the collection of point samples.

With the exception of analysing stations equipped with pricy facilities filtrating samples, none of these approaches can ensure concentration of samples at the time of their collection. In the case of low concentration in a flow and the need of more material for the analysis, we must either collect a very large water sample or execute the sample filtration manually at the time of its collection in the locality.

There are still complex stabile facilities used in biological research requiring high collection considerations in experimental river basins. They are, with their price and construction requirements, comparable with analysing stations monitoring water quality. The only simpler possibility is the method of sediment traps. We use an upright vessel of a defined neck area and let the materials from around flowing water sediments into it for a defined time. However, the use of these relatively simple devices is recommended and suitable only in stagnant waters, while their functioning in small water flows is not standard when the use is possible at all. When such a sediment trap is set up in the flow bed, it could be used in a relatively small water flow. However, we do not get a sample allowing the calculation of the theoretical material removal. If we wish to use the material further for biological, microbiological analyses respectively, the fast start of anoxic conditions, not allowing a longer exposure, could be also a problem. Another variant is a sediment trap of the form of a plastic box with net sides filled in with washed and sterilised sand. This arrangement allows for the concentration of samples (it catches flowing particles in spaces between sand grains even when the concentration is low in the water flow) as well as longer exposure, however, it asks for the complicated wash out of the sample from the sand and it provides a qualitative sample only.

The market does not offer any facilities allowing the collection of quantitative samples of fine suspended particles which could be applied, at the same time, in large series covering a larger area or for the parallel collection.

### DISCLOSURE OF INVENTION

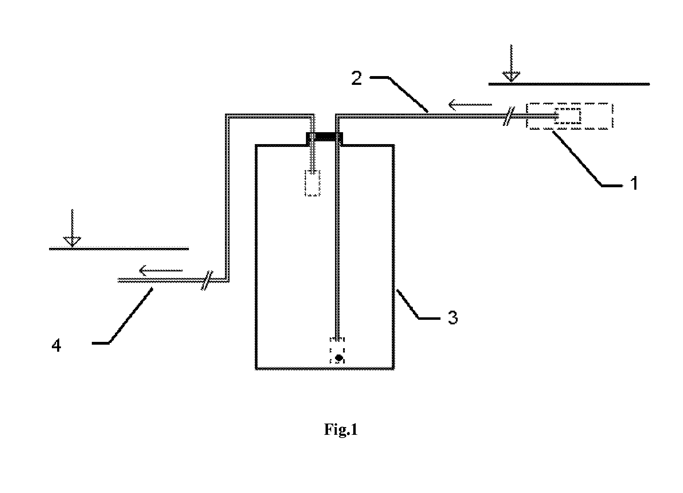

The continual sediment sampler for small sloping water flows (hereinafter called the "DDG sampler" only) uses the principle of connected vessels without the use of electricity. It features the water flowing into a sediment space, in which particles settle thanks to slower flows. Their settling speed is faster than the water flowing speed. Setting the difference between the intake hose height and the height of the outflow hose allows for the regulation of the flow through the facility - the flow conditions on the settling space and the character of the settling down particles. The figure 1 illustrates the function principle.

The facility starts with a suction basket equipped with an outside grill made of coated wire mesh not allowing larger flowing items in. The internal part of the suction basket is equipped with PE (polyethylene) mesh with defined size of holes - e.g. 1 mm. Only a fraction of fine particles of organic matter (FPOM) thus, for example, gets into the facility. There is a flexible pipe connecting the suction basket with the settling vessel. The intake part inside the settling vessel is equipped with an intake basket, with openings in its sides and bottom limiting short-circuiting, and the ballast keeping the intake basket always in the bottom part of the settling vessel with disregard to its position or fitting on an angled area. The settling vessel is the standardised water sampling bottle made of PET (polyethylene-terephtalate). The discharging pipe starts with the sucking part equipped with a mesh preventing the short-circuiting. Its end features a free tube with the outlet positioned at least 10 cm below the suction basket. The tested prototype was equipped with hoses of softened PVC (polyvinylchloride) having the inside diameter of 4 mm and the outside diameter of 6 mm and the settling vessel of the volume of 5 000 mL. It was catching and concentrated well even fine particles. Hoses of larger diameters and a smaller settling vessel can be used for catching of faster settling particles. The facility does not use electricity and it is thus independent from a power network or batteries.

The whole facility is put together on the basis of a kit principle. It consists of precisely manufactured plastic components and its individual parts are connected without the use of glue, screws or any other connecting materials. The settlement vessel and baskets are connected with flanges by a screw thread. Ends of individual hoses are cut at an angle for easier pulling through openings. The DDG sampler could be disassembled at any time without tools. It could be then cleaned and put together again even in field conditions. When some parts get damaged, they can be replaced.

The new DDG sampler always allows for the advantageous use in precise analyses and microbiological establishments in very clean waters or the cultivation of micro-organisms from floating materials, where the sterility must be maintained. The facility is thus suitable even for one time use. In common use, the DDG sampler could be washed out, or possibly chemically sterilised.

The construction of the facility allows, in necessary cases, its full submersion in the sampled environment. The sample liquid is led through the settling vessel in such a way that it washes the floating matter sample during the entire exposure and the oxygen mode and the chemical composition of the progressively collected sample are thus not changed. The thin-wall transparent construction maintains the thermal conditions of surrounding environments in the progressively collected samples. The similar light mode is also maintained, including the present ultra-violent part, which passes well through walls of the settling vessel made of PET. These functions are fundamental when collecting long-term samples for sensitive biological analyses of living parts in the floating matter and for chemical analyses of volatile substances.

The flow speed could be easily regulated with the facility by the height setting of the flexible discharging pipe in the place of its free outlet. The flow is thus not regulated with a throttle or a cock, where a blockage could occur during a long-term exposure.

### BRIEF DESCRIPTION OF DRAWINGS

Function principle is illustrated on Figure No. 1

### MADE FOR CARRING OUT THE INVENTION

The specific design example collecting fine organic matter in small flows
- Suction basket 1 with the outside PE coated wire mesh (the hole size 10 mm) of the shape of a flattened cylinder and the inside PE mesh (the hole size 1 mm) of the shape of a long cylinder (the diameter to length ratio is 1: 6),
- Suction pipe 2 of the length 2 000 mm (the outside diameter 6 mm, the inside diameter 4 mm - a transparent PVC hose),
- Intake PE basket with the ballast of washed quartz gravel and drilled openings in the bottom and sides (the diameter 5 mm), the settling vessel 3 - a PET bottle with fluted wall for better settling and the standard thread used in wider neck PET bottles,
- Discharge pipe 4 of the length 1000 mm covered by PE mesh (the hole site 1 mm) on the intake end and the shape of a cylinder (the diameter to length ratio is 1: 3) made of PVC hose with a free other end (the outside diameter 6 mm, the inside diameter 4 mm - a transparent PVC hose),
- The hose used in the construction must have a thick wall and be flexible to ensure tightness when going through openings, which have the diameter slightly smaller than the outside diameter of the hose, and, at the same time, to prevent accidental squeezing during its fitting in a flow - usual PVC hoses of the diameter as above correspond with these conditions while silicone ones are too soft.

The suction basket 1 is positioned in a flow in a place which is permanently flooded. It allows for changes of its shape and it could be thus adjusted to the specific micro relief. It is made stabile with local rocks or it could be fixed in the place in the clay, or sandy substrate, with an anchor of the shape of a long U made of strong PVC coated wire.

The settling vessel 3 is placed under the water surface and surrounded by stones, or anchored by a wire anchor. The position under the surface ensures the thermal conditions of the collected sample to be identical with conditions occurring in small water flows. The settling vessel could be placed at the height between the upper and lower working levels, or lower or higher, according to the specific situation in the place of collecting. The discharge pipe 4 is made stabile with rocks or a metal anchor fitted in the bottom. The pipe connecting the suction basket with the settling vessel and the discharge pipe are fixed in the water bed or placed under rocks. The described installation way makes the collecting facility almost invisible and that lowers the chance of someone damaging it. Thanks to the purchase costs, a theft of one or even a series of these facilities presents a reasonable risk during works undertaken in the field conditions.

A new or cleaned facility is filled in with water, without sediments, from the water flow before an installation, then, it is closed and left for pressure stabilisation. Then, the negative pressure at the end of the discharge pipe 4 puts the facility into operations. The water flow takes place on the principle of connected vessels, where the level above the placement of the suction basket is the upper level, while the level above the discharge pipe works as the lower level. The difference between the two levels is measured at the lifted up part of the discharge pipe. It is also the place, where we measure, with a graduated cylinder and a stop-watch, the actual flow through the facility. When measuring, the end of the discharge pipe must be at the level of the lower working water level in the water flow.

The flow is once again measured after the sampling period, before the collection of the sample. The facility allows for the option of the complete exchange of the settling vessel and the collection of the sample, or its proportional part, into a brought in sampling bottle. The DDG sampler works repeatedly well, when there are usual concentrations of sediments in oligotrophic flows, with the exposure periods up to 14 days, while collected samples are suitable for analyses in 24 hours. The facility works well when the flow is about 0.2 L/min.

When we experimentally verified series of prototypes, the DDG samplers set up in the described way in water flows of the 2^{nd} class survived even the flood flow Q1 (the flood flow repeated probably once a year). Some suction baskets were displaced or taken away, but all samples in settling vessels survived and could be consequently analysed.

Settling vessels allow for a lot of sediments in and that enables their use in longer exposures. The sediment does not have to be laboriously washed out and we know of the case when the flow through the facility was used for the calculation of substance removal. The facility can be placed also in very small water flows and, thanks to the small size of the suction basket it can work even during low flows. The whole facility can be removed after the exposure (the tested exposure periods were 24 hours, 7, 10, 14, and 24 days) or only the settling vessel, serving at the same time as a large volume sampling bottle, could be exchanged.

When the settling vessel is installed directly in a water flow, the facility maintains the oxygen conditions of the collected sample, the water chemical composition, the temperature and the exposure similar to the sampled environment even during long-term use. This function has been verified in the use of the facility variant described within the example of the design in oligotrophic water flows. When the high trophic environment, as contaminated rivers or waste waters, is sampled, the time of exposure must be shortened or the flow must be made faster to maintain the sample in optimal conditions.

## Claims

1. The mobile continual sediment sampler of wash loads in sloping water flows **characterized in that** a settling spacing vessel (3) of a cylindrical shape with a screw-on cap and two openings into which the suction and discharge pipes (2, 4) are fitted. The suction pipe is fitted in its intake part with a basket (1) and the outlet of the discharge pipe (4) must be at least 10 cm below the level of the suction basket (1)

2. Considering the requirement 1, the sampler **characterized in that** the suction basket (1) fitted with a mesh of optional size of holes

3. Considering the requirement 1, the sampler **characterized in that** the end of the discharge pipe which is equipped with an intake basket with openings and ballast

4. Considering the requirement 1, the sampler **characterized in that** the intake part of the discharge pipe equipped with a mesh of optional size of holes

5. Considering the requirement 1, the sampler **characterized in that** the end part of the discharge pipe the height of which can be adjusted

6. Considering the requirement 1, the sampler **characterized in that** individual components based on the kit principle

7. Considering the requirement 1, the sampler **characterized in that** the complete water resistance.
